Europäisches Patentamt

European Patent Office

Office européen des brevets

(11) Publication number: **0 088 368**
**A2**

(12) # EUROPEAN PATENT APPLICATION

(21) Application number: **83102071.4**

(22) Date of filing: **03.03.83**

(51) Int. Cl.³: **G 01 N 33/54, G 01 N 33/74**

(30) Priority: **05.03.82 JP 35315/82**
**05.03.82 JP 35314/82**

(43) Date of publication of application: **14.09.83**
**Bulletin 83/37**

(84) Designated Contracting States: **BE CH DE FR GB IT LI NL SE**

(71) Applicant: **Takeda Chemical Industries, Ltd., 27, Doshomachi 2-chome, Higashi-ku Osaka, 541 (JP)**

(72) Inventor: **Kondo, Koichi, 6-213, 2 Taishibashi 3-chome, Asahi-ku Osaka 535 (JP)**
Inventor: **Iwasa, Susumu, 21-2, Osumigaoka 1-chome Tanabe-cho, Tsuzuki-gun Kyoto 610-03 (JP)**
Inventor: **Ishikawa, Eiji, 24-1, Otsukadainishi 3-chome, Miyazaki 880-21 (JP)**

(74) Representative: **von Kreisler, Alek, Dipl.-Chem. et al, Deichmannhaus am Hauptbahnhof, D-5000 Köln 1 (DE)**

(54) **Immunochemical assay of human chorionic gonadotropin and reagent therefor.**

(57)  When an immunochemical assay of human chorionic gonadotropin (hCG) involving the use of an antibody supported on a carrier, an antigen and an antibody labeled with a labeling agent, in the proviso that said supported antibody and the labeled antibody are dissimilar antibodies which are not overlapping with each other in antigendeterminant position, that the supported antibody is an antibody specifically reactive to hCG, said labeling agent is β-D-galactosidase, and that said β-D-galactosidase is coupled with the corresponding antibody with N,N′-O-phenylenedimaleimide, is employed, a high reproducibility to hCG is obtained.

- 1 -

IMMUNOCHEMICAL ASSAY OF HUMAN CHORIONIC
GONADOTROPIN AND REAGENT THEREFOR

This invention relates to an immunochemical assay of human chorionic gonadotropin (hereinafter referred to as hCG).

Heretofore, the following two enzyme-immunoassay (hereinafter sometimes referred to briefly as EIA) methods have been proposed for the assay of hCG.

1)    Competitive method:

In the competitive method, a reagent containing a known quantity of hCG labeled with an enzyme and a sample containing an unknown quantity of hCG are subjected to a competitive coupling reaction with an anti-hCG antibody, then the enzymatic activity of the enzyme coupled with the antibody or of the enzyme not coupled with the antibody is measured, and the value found is compared with the value obtained similarly with a known quantity of hCG to estimate the quantity of hCG in the sample.

2)    Sandwich method:

In the sandwich method, a sample containing an unknown quantity of hCG is trapped to an anti-hCG antibody supported on a carrier, then an antibody labeled with an enzyme is trapped thereto and the enzymatic activity thereof is determined to estimate the quantity of hCG in the sample.

When the above competitive method 1) is practiced using a certain $\beta$-D-galactosidase as a labeling agent together with a certain antibody, a microassay of hCG is possible with high specificity (European Patent Application Publication No. 37,110). Regarding the sandwich method 2), the EIA using a supported antibody, an antigen and a labeled antibody permits an assay of hCG with high sensitivity, high accuracy and high specificity when the supported antibody and the labeled antibody are dissimilar antibodies not overlapping in antigen-determinant position (a particular region of an antigen at which an antigen reacts with an antibody at the combining site of the antibody) and one of said antibodies is specifically reactive to hCG (European Patent Application Publication No. 49,898).

However, by these methods, it is still difficult to determine hCG in very small quantities not exceeding about 0.1 to 2 mIU with sufficient accuracy and it was considered necessary to develop a more sensitive assay method for the purpose of ensuring an accurate diagnosis and monitoring of malignant tumors or elucidating the physiological role of hCG in healthy humans.

Under the foregoing circumstances, the present inventors conducted a further research and found that, in the EIA by the sandwich method using said specific antibody, the use of an antibody labeled with β-D-galactosidase results in a highly sensitive and highly accurate estimation of hCG which is present even in very small quantities. The finding was followed by further studies which have culminated in this invention.

This invention relates to (1) in an immunochemical assay of human chorionic gonadotropin involving the use of an antibody supported on a carrier, an antigen and an antibody labeled with a labeling agent, an improvement comprises in that said supported antibody and the labeled antibody are dissimilar antibodies which are not overlapping with each other in antigen-determinant position, that the supported antibody is an antibody specifically reactive to human chorionic gonadotropin, that said labeling agent is β-D-galactosidase, and that said β-D-galactosidase is coupled with the corresponding antibody employing N,N'-O-phenylenedimaleimide and (2) an immunochmemical assay kit for use in an assay of human chorionic gonadotropin which

- 3 -

comprises component 1) a reagent consisting of a conjugate which is prepared by coupling β-D-galactosidase with an antibody employing N,N'-O-phenylenedimaleimide and component 2) an antibody supported on a carrier, the antibody being not overlapping in antigen-determinant position with the antibody to be coupled with β-D-galactosidase and being specifically reactive to human chorionic gonadotropin.

Said invention is sometimes referred to as the invention (A).

In accordance with this invention, the antibody supported on a carrier and the antibody coupled with a labeling agent are two dissimilar antibodies which are not overlapping with each other in antigen-determinant position, and the antibody to be supported on the carrier is an antibody specifically reactive to hCG.

The carrier used in the present invention includes, among others, beads of gels {for example, agarose gel [e.g. Sepharose 4B, Sepharose 6B (Pharmacia Fine Chemicals, Sweden)], dextran gel [e.g. Sephadex G75, Sephadex G100, Sephadex G200 (Pharmacia Fine Chemicals)], polyacrylamide gel [e.g. Biogel P30, Biogel P60, Biogel P100 (Bio-Rad Laboratories, U.S.A.)]}, particles of cellulose [for example, Avicel (Asahi Kasei Inc. Japan), ion exchange cellulose (e.g. diethylaminoethyl-cellulose, carboxymethyl-cellulose)], physical adsorbents [for example, glass (e.g. glass beads, glass rods, aminoalkyl-glass beads, aminoalkyl-glass rods), silicone rubbers, styrene resin (e.g. polystyrene beads, polystyrene granules)], ion exchange resins {for example, weakly acid cation exchange resins [e.g. Amberlite IRC-50 (Rohm and Haas, U.S.A.), Zeocarb 226 (Permutit, West Germany)], weakly basic anion exchange resins [e.g. Amberlite IR-4B (Rohm and Haas), Dowex 3 (Dow Chemical, U.S.A.)]} and so on.

As examples of the antibody specifically reactive to hCG, there is mentioned (1) an antibody described in Endocrinology, Vol. 104 (1979), P. 396. Thus, an hCG-specific peptide at the C-terminal of hCG-β sub-unit and

- 4 -

a carrier protein such as bovine albumin or bovine-thyroglobulin are condensed in the presence of a watersoluble carbodiimide reagent such as 1-ethyl-3-(3-dimethyl-aminopropyl)carbodiimide, and using the resulting condensate and Freund's complete adjuvant or incomplete adjuvant, a warm-blooded animal such as rabbit is immunized a plurality of times to produce an antibody. This procedure gives an antiserum which reacts specifically to hCG.

(2) An hCG-specific anti-hCG antibody described in European Patent Application Publication No. 37,110 is mentioned. The antibody is prepared as follows: The peptide of the formula [I]:

$$\text{H-R-Pro-Ser-Asp-Thr-Pro-Ile-Leu-Pro-Gln-OH} \qquad \text{[I]}$$

[wherein R is a partial peptide of 1 to 14 amino acid residues including 14-Gly of the peptide $Ala^1-Pro^2-Pro^3-Pro^4-Ser^5-Leu^6-Pro^7-Ser^8-Pro^9-Ser^{10}-Arg^{11}-Leu^{12}-Pro^{13}-Gly^{14}$],

as immobilized on a carrier, is contacted with a body fluid containing an anti-hCG antibody, and the anti-hCG antibody specifically absorbed is separated by elution.

(3) Furthermore, as an antibody which reacts specifically with hCG, there may be mentioned an antibody prepared by condensing a peptide of the formula [I] mentioned above with a carrier protein in the presence of glutaraldehyde (hereafter briefly, GLA), inoculating a warm-blooded animal other than man with the resulting condensate to produce an antibody and recovering the same (European Patent Application Publication No. 49,898).

The carrier protein referred to above is a substance which is used as coupled with a hapten (a substance of low molecular weight) for producing an antibody to the hapten such as a peptide which, as it is alone, cannot induce formation of an antibody, and includes such proteins as bovine serum albumin, bovine gamma-globulin, bovine thyroglobulin, tetanus toxoid, hemocyanin and polyamino acid.

The coupling of the peptide of the formula [I] with

- 5 -

a carrier protein in the presence of GLA can be conducted by the conventional method [e.g. "Hormone and Metabolic Research", Vol, 8 (1976) P. 241]. The relative amount of peptide [I] and carrier protein is preferably about 1:1 to 2:1, and the reaction pH of about 7.3 gives satisfactory results in many cases. The reaction time is somewhere between about 2 and 6 hours and a reaction time of about 3 hours is usually appropriate. The condensation product thus obtained is dialyzed against water of about 4°C, lyophilized and stored as usual.

The condensation product thus obtained is used to inoculate a warm-blooded animal other than man.

The warm-blooded animal other than man, which is used in the production of the above hCG-specific antibody, includes, among others, mammarian warm-blooded animals (e.g. rabbit, sheep, rat, mouse, guinea pig, cattle, horse, pig) and avian species (e.g. chicken, pigeon, duck, goose, quail).

To inoculate the condensation product into such a warm-blooded animal other than man, the condensation product is used in an amount sufficient to produce the desired antibody. For example, 2 mg per dose of the condensation product is emulsified with equal volumes (1 ml) of physiological saline and Freund's complete adjuvant and the emulsion is injected subcutaneously into dorsal sites and rear foot pads of a rabbit for a total of 5 times at intervals of 4 weeks. The above procedure yields the desired antibody in many instances.

The antibody thus produced in the body of the warm-blooded animal can be harvested as follows. Thus, in the case of a rabbit, for instance, blood is withdrawn from the ear vein and centrifuged to separate the serum normally at a time between 7 to 12 days after the last immunization.

The carrier for supporting the antibody used as immobilized thereon in the assay of hCG may be any of the carriers mentioned above.

The coupling of the carrier with the antibody or antigen may be effected by the conventional method. For example, the cyanogen bromide method as well as the GLA method described in "Metabolism and Disease", Vol, 8 (1971), P. 696 published by Nakayama Shoten Inc., Japan may be mentioned. As a more expedient procedure, the antibody may be physically adsorbed on the surface of the carrier.

As examples of the peptide of the formula [I], there is mentioned a C-terminal fragment peptide of hCG-β (123-145)(hereinafter abbreviated to "peptide (I)") represented by the formula: H-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH. The fragment is produced, for example, by a method described in European Patent Application Publication No. 37,110.

Throughout this specification, when abbreviations are used to amino acids, peptides, etc., they are either the abbreviations according to IUPAC-IUB Commission on Biochemical Nomenclature or those of common use in this field of art. The following is a partial list of the abbreviations. It is to be understood that when optical isomers exist in regard to amino acids, etc., L-forms are meant unless otherwise indicated.

Ala : alanine
Pro : proline
Ser : serine
Leu : leucine
Arg : arginine
Gly : glycine
Asp : aspartic acid
Thr : threonine
Ile : isoleucine
Gln : glutamine

The various peptides which are employed for the production of the specific antibody according to the present invention can be produced by procedures known

- 7 -

per se.  While both of the solid-phase and the liquid-phase methods of synthesis may be employed, the latter method is more often advantageous.  Such methods for peptide synthesis include those described in the literature, e.g. Schröder and Lubke:  The Peptides, Vol. 1 (1966), Academic Press, New York, U.S.A. and Izumiya et al.: "Peptide Gosei" (Peptide Synthesis) (1975), Maruzen Inc., Japan, namely the azide method, chloride method, acid anhydride method, mixed acid anhydride method, DCC method, active ester method, Woodward Reagent K method, carbodiimidazole method, reduction-oxidation method, DCC-additive (e.g. HONB, HOBt, HOSu) method and so on.

The antibody in the labeled antibody used in the invention (A) is an antibody which reacts nonspecifically to hCG and is not overlapping with the supported antibody in antigen-determinant position.

An example of the antibody nonspecifically reactive to hCG is the antibody which shows cross-reactivity with other structurally analogous protein hormones such as hLH (human luteinizing hormone), which antibody may be prepared by inoculating a warm-blooded animal other than a human being with a purified hCG derived from human urine in the conventional manner to produce an anti-hCG antibody, subjecting the same antibody to salting-out to recover γ-globulin fraction, subjecting the same fraction to affinity chromatography on a column packed with a solid phase coupled with the C-terminal peptide of hCG-β to recover an effluent and purifying the effluent further by affinity chromatography on a column of a solid phase with which hCG has been coupled.

To couple the labeling agent β-D-galactosidase with the antibody, N,N'-O-phenylenedimaleimide (hereinafter sometimes referred to briefly as PDM) is employed.

In a typical method of effecting this coupling, an antibody Fab' fragment as prepared by the method of Kato et al. [The Journal of Immunology 116 (1976), P. 1554] is reacted with PDM in a buffer at pH about 5 to 8 at a temperature of about 0°C to 40°C for a period of 10 minutes to 24 hours. Examples of the buffer include 0.1 M sodium acetate buffer (pH 5.0) and 0.1 M phosphate buffer (pH 5.8). To react this maleimidated antibody Fab' fragment with β-D-galactosidase, the two reagents are incubated in a buffer at a temperature of about 0°C to 40°C for a period of about 10 minutes to 48 hours. An example of the buffer mentioned above is 0.1 M phosphate buffer containing 1 mM of sodium ethylenediaminetetraacetate (EDTA) (pH 6.5).

A specific exemplary procedure for coupling β-D-galactosidase with an antibody employing N,N'-O-phenylenedimaleimide is described below.

The anti-hCG antibody IgG or the $F(ab')_2$ fraction obtained by pepsin degradation is reduced in the presence of a mercaptoethylamine and the unreacted material is removed by gel filtration. The resultant anti-hCG antibody IgG or Fab' is reacted with N,N'-O-phenylenedimaleimide, followed by removal of low-molecular fractions by gel filtration. The resultant maleimidated antibody is reacted with β-D-galactosidase and the reaction product is purified by gel filtration to give a conjugate of β-D-galactosidase with either anti-hCG antibody IgG or Fab'.

The assay method according to the invention (A) will be described in further detail below.

First, (1) a sample containing hCG is added to said antibody supported on a carrier to conduct an antigen-antibody reaction and, then, the above-mentioned conjugate of β-D-galactosidase with anti-hCG antibody IgG or Fab' is further reacted.

Examples of the hCG-containing sample used in the enzyme-immunoassay method according to the invention (A) include such biological fluids as urine, serum, plasma, cerebrospinal fluid, etc. and various organ extracts. Particularly, urine, serum and plasma are generally assayed.

(2) To the reaction product obtained as above in (1)

- 9 -

is added a substrate for β-D-galactosidase, e.g. 4-methyl-umbelliferyl-β-D-galactoside and the liberated 4-methyl-umbelliferone, for instance, is determined with a fluorophotometer to estimate the enzymatic activity of the above-mentioned reaction product.

(3) The above procedures (1) and (2) are applied to standard solutions containing known quantities of hCG to construct a calibration curve by plotting the fluorescence intensity reading against the amount of hCG.

(4) The fluorescence intensity of the sample containing an unknown quantity of hCG is compared with the calibration curve to estimate the hCG content of the sample.

The assay kit for the immunochemical assay of hCG by the sandwich method in accordance with the invention (A) comprises:

(1) The antibody immobilized on a carrier [component 1)],

(2) The antibody labeled with β-D-galactosidase [component 2)],

(3) The standard hCG of about 0 to 100 IU,

(4) The buffer for dilution of reagents (2) to (3) and the test fluid. (This may be any buffer which can be used for diluting these reagents and test fluid: Phosphate buffer or glycine buffer at pH about 6 to 9 may be mentioned as examples.)

(5) The buffer for use in the washing of the carrier after incubation. (This may be any buffer that can be used for washing the carrier: Phosphate or glycine buffer may be mentioned as examples.)

(6) The substrate (preferably 4-methylumbelliferyl-β-D-galactoside, o-nitrophenyl-β-D-galactoside or p-nitrophenyl-β-D-galactoside), the buffer for dissolving the substrate (preferably, phosphate buffer) and the buffer for terminating the enzymatic reaction (preferably, carbonate buffer or glycine buffer).

The above kit is preferably used in the following manner.

About 10 to 200 μl of the standard hCG or test fluid

- 10 -

is diluted with reagent (4), followed by addition of a known amount of reagent (1). The mixture is reacted at 0 to 40°C for about 1 to 24 hours. The carrier is washed with reagent (5) and, then, about 10 to 300 μl of reagent (2) is added thereto and the reaction is conducted at 0 to 40°C for about 1 to 24 hours. Then, the carrier is washed with reagent (5), and the activity of labeling agent combined to the carrier is measured. Thus, about 10 to 1000 μl of the substrate solution is added, the reaction is conducted at 20 to 40°C for about 0.5 to 24 hours, the enzymatic reaction is then terminated, and the absorbancy or fluorescence intensity of the reaction mixture is determined.

By the assay method according to the invention (A), hCG can be assayed with high specificity and sensitivity.

Since the invention (A) ensures accurate assays of hCG with high sensitivity and without interferences from other analogous hormones (e.g. human luteinizing hormone), it provides a very useful means of diagnosis and prognostic management of chorionic tumor and other hCG-producing tumors.

On the other hand, a method of eliminating an interference of non-specific reaction in a specific immunological assay of hCG was found.

hCG is a kind of protein hormone produced from the chorionic cells which are formed simultaneously with conception and promotes secretion of progesteron. While the assay of hCG has been commonly employed for an early diagnosis of pregnancy, hCG-producing malignant tumors as well as chorionic tumor have also been discovered in recent years. Thus, hCG has been detected in the urine, blood or/and cerebrospinal fluid of tumor-bearing patients. Further, hCG has also been detected in various organs and blood of healthy humans. It has, therefore, come to be recognized that a qualitative and quantitative determination of this hormone is of significance in the diagnosis and prognosis of malignant tumors and in studies on the physiological role of the hormone in healthy humans. However, the diagnosis of such tumors requires an assay of trace quantities of hCG in the range of 1 to 10 mIU or less, and what matters in the assay is the immunological cross-reaction with hLH which is structurally analogous with hCG.  Since the physiological hLH concentration may be as high as 100 to 150 IU/ℓ in the urine and 200 to 300 IU/ℓ in the blood, it is necessary to immunologically differentiate hCG from hLH in order to estimate the hCG content of a body fluid or an organ.

Under the circumstances, for the purpose of constructing a hCG-specific antibody which would not cross-react with hLH, a C-terminal peptide of hCG-β which has an amino acid sequence not existing in hLH was synthesized and an antibody reactive to such a peptide was prepared.  It was found that an immunological assay using this antibody as supported on a carrier permits an accurate estimation of hCG (European Patent Application Publications Nos. 37,110 and No. 49,898).  However, even with such method it was found that when the quantity of hCG is as small as about 0.1 to 2 mIU or less, the concomitant hLH is non-specifically adsorbed on the antibody-bound solid phase to interfere with an exact specific estimation of hCG.

The inventors conducted further studies and found that the addition of a serum and a proteinous substance such as albumin to a buffer of the immunoassay inhibits the non-specific adsorption of hLH on the antibody-bound solid phase.  The finding was followed by further research which has culminated in the establishment of an invention.

Such invention relates to (1) in an immunochemical assay of hCG involving the use of an antibody supported on a carrier, an antigen and an antibody labeled with a labeling agent, an improvement is that a serum and a proteinous substance are added to a reaction system comprising a sample fluid containing an unknown quantity of the

- 12 -

antigen and the carrier-supported antibody, and (2) an immunochemical reagent for assay of hCG which contains a serum and a proteinous substance. Said invention is referred to as the invention (B). As the carreir of the carrier-supported antibody, thére is mentioned one similar to those described in the invention (A).

The antibody to be supported on a carrier and the antibody to be labeled with a labeling agent may each be either an antibody which reacts non-specifically with hCG or an antibody which reacts specifically with hCG.

The antibody which reacts non-specifically with hCG may, for example, be an antibody which shows cross-reactivity with structurally analogous protein hormone such as hLH as prepared in the following manner. Thus, hCG as purified from human urine in the conventional manner is used to inoculate a warm-blooded animal other than a human being to produce an anti-hCG antibody, and after salting-out, the resulting γ-globulin fraction is subjected to affinity chromatography on a column packed with a solid phase carrying a C-terminal peptide of hCG-β as bound thereto. The effluent from the column is further purified by affinity chromatography on a column packed with a solid phase carrying hCG as bound thereto to give the desired non-specific antibody.

As the antibody specifically reactive to hCG, there is mentioned one similar to those used in the invention (A), and as the method of producing the antibody, there is mentioned one similar to those described in the invention (A).

The coupling of the carrier with the antibody may be effected by the conventional method. For example, the cyanogen bromide method as well as the GLA method described in "Metabolism and Desease", Vol. 8 (1971), P. 696 may be mentioned. As a more expedient procedure, the antibody may be physically adsorbed on the surface of the carrier.

- 13 -

The labeling agent for said labeled antibody which is used in the assay of hCG includes, for instance, radioisotopes, enzymes, fluorescent substances and luminous substances. Examples of such radioisotopes are $^{125}I$, $^{131}I$, $^{3}H$ and $^{14}C$. The enzyme is desirably one which is stable and has a high specific activity. Thus, there may be mentioned, for example, (1) carbohydrase [for example, glycosidase (e.g. β-D-galactosidase, β-glucosidase, β-glucuronidase, β-fructosidase, α-galactosidase, α-glucosidase, α-mannosidase), amylase (e.g. α-amylase, β-amylase, isoamylase, glucoamylase, Taka-amylase A), cellulase, lysozyme], (2) amidase (e.g. urease, asparaginase), (3) esterase [for example, cholinesterase (e.g. acetylcholinesterase), phosphatase (e.g. alkaline phosphatase), sulfatase, lipase], (4) nuclease (e.g. deoxyribonuclease, ribonuclease), (5) iron-prophyrin enzymes (e.g. catalase, peroxidase, cytochrome oxidase), (6) copper enzymes (e.g. tyrosinase, ascorbic acid oxidase), (7) dehydrogenase (e.g. alcohol dehydrogenase, malic acid dehydrogenase, lactic acid dehydrogenase, isocitric acid dehydrogenase), etc. The fluorescent substance may for example be fluorescamine, fluorescence isothiocyanate, etc. and the luminous substances include, among others, luminol, luminol derivatives, luciferin, lucigenin, etc. Among the labeling agent, β-D-galactosidase is most preferable.

The coupling of the hCG-specific antibody with the labeling agent can be effected by the conventional method, e.g. the chloramine T method [Nature, 194 (1962), P. 495], periodic acid method [Journal of Histochemistry and Cytochemistry 22 (1974), P. 1084], maleimide method [Journal of Biochemistry 79 (1976), P. 233, Journal of Immunology 116, (1976), P. 1554].

The samples to be tested by the immunoassay method according to the invention (B) may be any samples from organisms, such as urine, serum, plasma, spinal fluid and organ extracts. Among them, urine, serum and plasma are

- 14 -

used frequently.

The serum employed in accordance with the invention (B) may be any serum that does not contain hCG, and some preferred sera are those of sheep, rabbits and calves. Human sera from which hCG was removed by affinity column chromatography may also be advantageously utilized. Such a serum is added to the immunoassay system in a proportion of about 2 to 40% (v/v) and preferably, of about 10 to 30% (v/v) as a final concentration.

The proteinous substance employed in accordance with the invention (B) may for example be a serum protein (e.g. albumin, globulin, etc.) or a highly hydrophobic protein (e.g. gelatin, collagen hydrolysate, etc.). A preferred example is bovine serum albumin. Such a proteinous substance is added to the immunoassay system in a fairly large proportion of about 0.3 to 5% (w/v) and, preferably, of about 0.5 to 2% (w/v) as a final concentration.

To conduct the specific immunochemical assay of hCG of the invention (B), the sample fluid containing unknown amount of hCG is first reacted with a solid phase with which the antibody has been coupled either chemically or physically by the conventional procedure. In this reaction system, a serum and a proteinous substance are added to the buffer solution (first reaction). Then, after the solid phase is washed, a known quantity of the labeled antibody is added and reacted (second reaction). Then, usually, the solid phase is washed well and the activity of the labeling agent which is bound to the solid phase is determined. When the labeling agent is a radioisotope, its activity is measured with a well counter or a liquid scintillation counter. When the labeling agent is an enzyme, a substrate is added, the mixture is allowed to stand and the enzymatic activity is assayed colorimetrically or fluorometrically. In case the labeling agent is a fluorescent substance or a luminous substance, these are

- 15 -

measured by the conventional methods. In the above assay procedure, the washing of the solid phase as an intermediate process between the first reaction and the second reaction may be omitted or, for further simplification, the sample fluid, antibody-bound solid phase and the labeled antibody may be simultaneously added and reacted.

In accordance with the invention (B), the concomitant presence of both a serum and a proteinous substance leads to an improved inhibition of non-specific hLH adsorption.

In the invention (B), hLH which may be present in a sample fluid is not bound to the carrier-supported antibody, the hCG which is present in a trace quantity in the sample fluid reacts specifically with the said antibody, with the result that the trace quantity of hCG present in the sample fluid is accurately estimated. The invention (B) thus provides a very useful means for the diagnosis and prognostic management of chorionic tumor and other hCG-producing tumors.

As described above, the immunoassay method of the invention (B) permits an accurate assay of a trace quantity of hCG in a biological fluid sample to the exclusion of hLH and, thus, ensures an increased reliability of EIA and other hCG assays while permitting a specific estimation of hCG in the presence of hLH.

Moreover, a buffer containing both a serum and a proteinous substance as prepared in the described manner is a useful component of a hCG diagnostic reagent and is of use as a component of RIA (radioimmunoassay), EIA or the like kit.

The assay kit for the immunochemical assay of hCG in accordance with the invention (B) comprises:

(1) The antibody immobilized on a carrier,

(2) The antibody labeled with a labeling agent,

(3) The standard hCG of about 0 to 10 IU,

(4) The buffer for dilution of reagents (2) to (3) and the test fluid. [There may be mentioned phosphate buffer or glycine buffer at pH about 6 to 9, which contains about 10% sheep serum as a serum and about 1% bovine serum albumin (hereinafter referred to as BSA) as a proteinous substance.]

(5) The buffer for use in the washing of the carrier after incubation. (This may be any buffer solution that can be used for washing the carrier; phosphate or glycine buffer may be mentioned as an example.)

(6) When the labeling agent is an enzyme, the reagents used in measuring the activity of the enzyme. For example, when the enzyme is β-D-galactosidase, the substrate (preferably 4-methylumbelliferyl-β-D-galactoside or o-nitrophenyl-β-D-galactoside), the buffer for dissolving the substrate (preferably, phosphate buffer) and the buffer for terminating the enzymatic reaction (preferably, carbonate buffer or glycine buffer). When a fluorescent substance is used as the labeling agent, the materials for measuring the intensity of fluorescence. When a luminous substance is used at the labeling agent, for example, in the case of luminol, the oxidizing agent (preferably, hyrogen peroxide), the catalyst (preferably, micro-peroxidase or a hypochlorite) and the buffer for dissolving the oxidizing agent as well as the catalyst (preferably, sodium hydroxide solution or carbonate buffer) are used.

It should be understood that (1) and (2) may be a premix.

The above kit is preferably used in the following manner.

About 10 to 200 μl of the standrad hCG or test fluid is diluted with reagent (4), followed by addition of a known amount of reagent (1). The reaction is conducted at about 0 to 40°C for about 1 to 24 hours. After washing the carrier with reagent (5), about 10 to 300 μl of reagent (2) is added to the carrier and the reaction is conducted at about 0°C to 40°C for about 1 to 24 hours. Then, the

- 17 -

carrier is washed with reagent (5) and the activity of labeling agent combined to the carrier is measured. When the labeling agent is a radioisotope, a well counter or a liquid scintillation counter is employed. When the labeling agent is an enzyme, about 10 to 1000 µl of the substrate solution is added, the reaction is conducted at about 20 to 40°C for about 0.5 to 24 hours, the enzymatic reaction is then terminated, and the absorbancy or fluorescence intensity of the reaction mixtuer is determined.

Brief description of drawings:

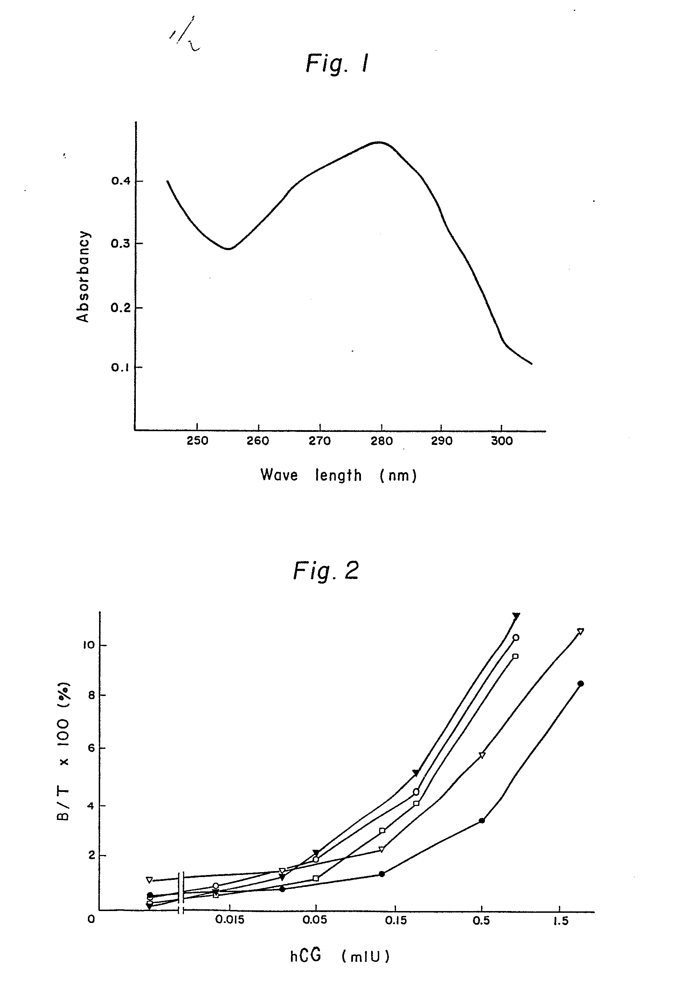

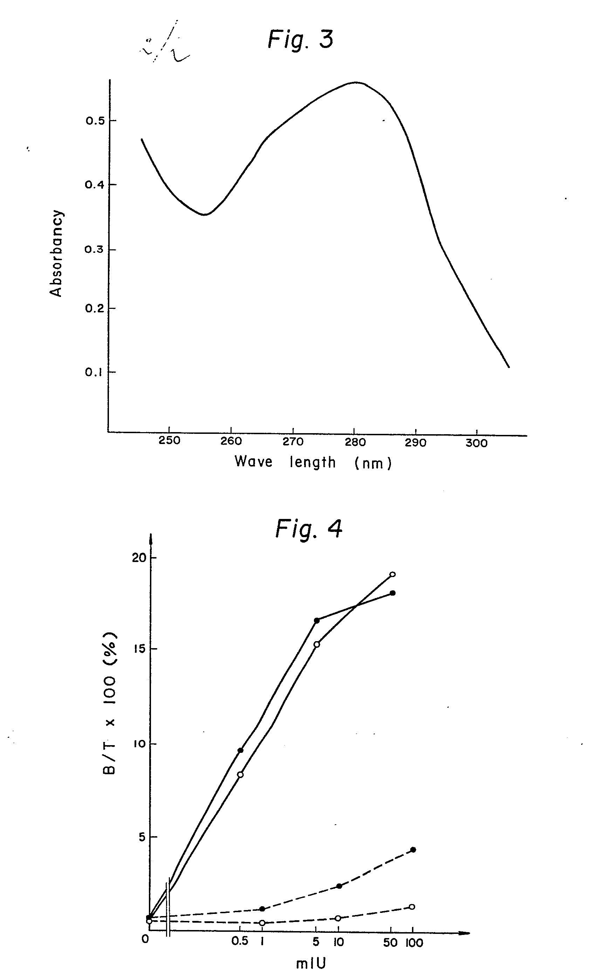

Figs. 1 and 3 show the ultraviolet absorption specta of the anti-hCG antibody Fab'-β-D-galactosidase conjugate obtained in Example 1 and anti-hCG antibody IgG-β-D-galactosidase conjugate obtained in Reference Example 7, respectively. Fig. 2 shows standard curves of hCG, in which -o- represents the result of Example 1; -▢- represents the result of Example 2; -▼- represents the result of Example 3; -▽- represents the result of Reference Example 7; and -●- represents the result of Reference Example 8. Fig. 4 shows a standard curve of hCG and non-specific adsorption of hLH obtained in Reference Example 10. In "B/T x 100" indicated in the ordinate of the Figs. 2 and 4, B means the enzymatic activity bound to the solid phase, and T means the total enzymatic activity added to the assay tube.

- 18 -

The following Examples and Reference Examples are given to illustrate the inventions (A) and (B) in further detail.

Reference Example 1

Production of anti-hCG antibody (anti-hCG serum):

In 1 ml of physiological saline was dissolved 1 mg of hCG-β prepared by the method described in Endocrinology 88, (1971), p. 1045, and 1 ml of Freund's complete adjuvant [Tachibana et al.: Men-eki-no-Seikagaku (Biochemistry of Immunity), p. 26, Kyoritsu Shuppan Inc. Japan (1967)] was added and stirred well to prepare an emulsion. This emulsion was injected into the bilateral femoral muscles and subcutaneously at several dorsal sites of a rabbit. The above procedure was repeated at intervals of 3 weeks for a total of 5 times and a blood sample was taken one week after the last immunization for a pilot assay. In this manner, there was obtained an anti-serum N305B having an affinity also for the C-terminal fragment peptide (I) of hCG-β.

Reference Example 2

Production of specific anti-hCG antibody:

Five (5) mg of peptide (I) was dissolved in 8 ml of 0.1 M NaHCO$_3$ containing 0.5 M NaCl. To this solution was added 1 g of BrCN-activated Sepharose 4B previously washed with 1/1,000 N HCl. The mixture was stirred at 5°C overnight. Then, the Sepharose was washed well with the 0.1 M NaHCO$_3$ solution containing 0.5 M NaCl as used above, followed by addition of 10 ml of 0.5 M ethanolamine adjusted to pH 8 with hydrochloric acid. The reaction was conducted at room temperature for one hour, after which time the Sepharose was washed with (1) 0.1 M acetate buffer containing 1 M NaCl (pH 4.0), (2) 0.1 M borate buffer containing 1 M NaCl (pH 8.0) and (3) 0.02 M borate buffer containing 0.15 M NaCl (pH 8.0) in the order mentioned. The Sepharose was then packed into a column.

Eight (8) ml of the anti-hCG serum N305B obtained according to Reference Example 1 was subjected to fractional precipitation with 1.5 g of anhydrous sodium sulfate and the resultant γ-globulin fraction was passed through the above column of peptide (I)-Sepharose 4B (column size: 0.9 × 4 cm).

The column was washed with 0.02 M borate buffer containing 0.15 M NaCl (pH 8.0) to remove the anti-hCG antibodies showing cross-reactivity with hLH, hFSH (human follicle-stimulating hormone) and hTSH (human thyroid-stimulating hormone). Then, elution was carried out with 0.17 M glycine-HCl buffer (pH 2.3) to recover the specific anti-hCG antibody N305BS having a strong affinity for the C-terminal fragment peptide (I) of HCG-β (Protein content 1.2 mg).

### Reference Example 3

Production of anti-hCG-β C-terminal fragment peptide (I) antibody:

In 4 ml of 0.2 M phosphate buffer (pH 7.3) were dissolved 25 mg of the C-terminal peptide (I) of hCG-β and 50 mg of bovine thyroglobulin (briefly, BTG), followed by addition of 4 ml of 5% aqueous GLA. The mixture was stirred at room temperature for 3 hours, after which it was dialyzed against water at 4°C (2 ℓ of water × 4) and lyophilized to obtain an immunogen. In 0.75 ml of physiological saline was dissolved 1.5 mg of the above hCG-β C-terminal peptide (I)-BTG conjugate, followed by addition of 0.75 ml of Freund's complete adjuvant. The mixture was stirred well to prepare an emulsion. The emulsion was injected intramuscularly into bilateral femoral muscles and subcutaneously several dorsal sites of a rabbit. The above procedure was repeated four times at intervals of 4 weeks and the blood was collected a week after the last immunization, centrifuged to separate the antiserum. In the above manner, anti-hCG-β C-terminal peptide (I) serum N313B was obtained.

- 20 -

This antiserum N313B was precipitated with ammonium sulfate in the conventional manner and the resulting γ-globulin fraction was applied to a Sepharose 4B column (0.9 cm dia. × 4 cm long) carrying 2 mg of hCG.

The column was washed with 0.02 M borate buffer containing 0.15 M NaCl (pH 8.0) and elution was carried out with 0.17 M glycine-HCl buffer (pH 2.3), whereby a specific antibody N313BS having a high affinity for hCG was obtained.

Reference Example 4

Preparation of a non-specific anti-hCG antibody:

The antiserum N305B obtained in Reference Example 1 was precipitated with ammonium sulfate and subjected to affinity chromatography on a column (0.9 cm in dia., 4 cm long) of Sepharose 4B carrying 5 mg of the C-terminal peptide (I) of hCG-β. An antibody fraction was recovered as the effluent. This antibody fraction was passed through a column (0.9 cm in dia., 4 cm long) of Sepharose 4B carrying 2 mg of hCG. The column was washed with a 0.02 M borate buffer (pH 8.0) containing 0.15 M NaCl and, then, elution was carried out with 5 M $MgCl_2$ to recover a non-specific antibody N305BG having a high affinity for hCG.

Reference Example 5

Preparation of a solid phase carrying an antibody:

(1)  To 1500 polystyrene balls (3.2 mm in dia., Precision Plastics Ball Co., Chicago, U.S.A.) was added 30 ml of a solution (30 µg/ml) of the antibody N305BS, or N305BG which was prepared in Reference Example 2 or 4 in 0.01 M NaCl-0.01 M phosphate buffer (pH 8.0) and the mixture was incubated at 5°C overnight. The polystyrene balls were washed with 0.05 M phosphate buffer (pH 7.0) containing 0.1% bovine serum albumin (BSA) and stored in the cold. This is used as a solid phase carrying the antiboty.

(2)  To 1500 polystyrene balls (3.2 mm in dia., Precision Plastics Ball Co., Chicago, U.S.A.) was added 30 ml of a solution (30 µg/ml) of the antibody N313BS, which was prepared in Reference Example 3, in 0.01 M NaCl-0.01 M phosphate buffer (pH 8.0) and the mixture was incubated at 5°C overnight. The polystyrene balls were washed with 0.05 M phosphate buffer (pH 7.0) containing 0.1% BSA and stored in the cold. This is used as a solid phase carrying the antibody.

### Reference Example 6

Production of anti-hCG-β antibody and preparation of a solid phase carrying the antibody:

hCG-β was prepared according to the method of Morgan [Endocrinology, 88, (1971), p. 1045].

In 2 ml of 0.1 M phosphate buffer (pH 7.0) were dissolved 5 mg of hCG-β and 20 mg of BTG, followed by addition of 0.5 ml of 1% aqueous GLA. The mixture was stirred at room temperature for 3 hours, after which it was dialyzed against water at 4°C (2 ℓ of water x 4) and lyophilized to obtain an immunogen. In 0.75 ml of physiological saline was dissolved 1.5 mg of the above hCG-β-BTG conjugate, followed by addition of 0.75 ml of Freund's complete adjuvant. The mixture was stirred well to prepare an emulsion. The emulsion was injected intramuscularly into bilateral femoral muscles and subcutaneously several dorsal sites of a rabbit. The above procedure was repeated four times at intervals of 4 weeks and the blood was collected a week after the last immunization, centrifuged to separate the antiserum. In the above manner, anti-hCG-β serum N307B was obtained.

This antiserum N307B was precipitated with ammonium sulfate in the conventional manner and the resulting γ-globulin fraction was applied to a Sepharose 4B column (0.9 cm dia. x 4 cm long) carrying ~~2 mg of hCG.~~ *peptide* (I).

The column was washed with 0.02 M borate buffer containing 0.15 M NaCl (pH 8.0) and elution was carried out with 0.17 M glycine-HCl buffer (pH 2.3), whereby a specific antibody N307BS having a high affinity for hCG was obtained.

To 500 polystyrene balls (4.8 mm in dia., Precision Plastics Ball Co., Chicago, U.S.A.) was added 30 ml of a solution (15 μg/ml) of the antibody N307BS, which was prepared in Reference Example 6 in 0.01 M NaCl-0.01 M phosphate buffer (pH 8.0) and the mixture was incubated at 5°C overnight. The polystyrene balls were washed with 0.05 M phosphate buffer (pH 7.0) containing 0.1% bovine serum albumin (BSA) and stored in the cold. This is used as a solid phase carrying the antibody.

- 23 -

Example 1

Quantitative determination of hCG with a β-D-galactosidase-anti-hCG rabbit antibody N305BG (Fab') conjugate:

(1) Preparation of a β-D-galactosidase-anti-hCG rabbit antibody N305BG (Fab') conjugate:

To 5 mg of the antibody N305BG prepared in Reference Example 4 was added 0.1 mg of pepsin and the reaction was conducted at 30°C overnight. The reaction mixture was purified with a Sephadex G-150 column (2.5 cm in dia., 55 cm long). The resulting antibody $F(ab')_2$ fraction was reduced with β-mercaptoethylamine and the excess reagent was removed by passage through a column of Sephadex G-25 (0.9 cm in dia., 55 cm long). Then, 2 ml of a saturated solution of PDM was added and the reaction was conducted at 30°C for 30 minutes. The reaction mixture was purified with a Sephadex G-25 column (2.5 cm in dia., 55 cm long). To the resulting maleimidated Fab' was added 50 μl of a solution of β-D-galactosidase (5 mg/ml) and the mixture was reacted at 5°C overnight. After completion of the reaction, it was purified by Sepharose 6B column chromatography using a 0.02 M phosphate buffer (pH 6.5) containing 0.1% BSA, 0.1% $NaN_3$, 1 mM $MgCl_2$ and 0.1 M NaCl to recover a fraction containing enzymatic activity and antibody activity. This was a β-D-galactosidase-anti-hCG antibody N305BG (Fab') conjugate.

The physical properties of this conjugate are as follows.

1) This conjugate degrades 4-methylumbellipheryl-β-D-galactoside, o-nitrophenyl-β-D-galactoside and p-nitrophenyl-β-D-galactoside, which are synthetic substrate for EIA, to liberate 4-methylumbellipherone, o-nitrophenol and p-nitrophenol, respectively.

2) When 4-methylumbellipheryl-β-D-galactoside is used as a synthetic substrate, the Michaelis' constant of this conjugate is the same as the value of the original β-D-galactosidase.

3) The optimal pH of enzymatic activity is 6.5 to 7.3.

4) At least about 90% of this conjugate having enzymatic activity is reactive to hCG and its antibody activity and enzymatic activity are both stable in a refrigerator for at least 6 months.

5) The conjugate has a molecular weight of about 650,000 and its molar antibody Fab'/enzyme ratio is about 2.0.

6) The conjugate is readily soluble in aqueous solvents at pH 5 to 9.

7) The ultraviolet absorption spectrum of the conjugate is shown in Fig. 1.

8) Its amino acid analysis is shown in Table 1.

Table 1

| Amino acid | β-D-Galactosidase-anti-hCG rabbit antibody N305BG (Fab') conjugate |
| --- | --- |
| Lys (lysine) | 111 |
| Arg | 94 |
| Asp | 166 |
| Thr | 119 |
| Ser | 110 |
| Glu (glutamic acid) | 215 |
| Pro | 87 |
| Gly | 100 |
| Ala | 133 |
| Val (valine) | 114 |
| I-Leu | 52 |

| Amino acid | β-D-Galactosidase-anti-hCG rabbit antibody N305BG (Fab') conjugate |
|---|---|
| Leu | 159 |
| Tyr (tyrosine) | 39 |
| Phe (phenylalanine) | 69 |

(Note): The number of moles of each amino acid per 100 moles of glycine.

(2) Quantitative determination of hCG with a β-D-galactosidase-anti-hCG rabbit antibody N305BG (Fab') conjugate:

To 150 µl of Buffer A [a 0.02 M sodium phosphate buffer (pH 7.0) containing 0.1 M NaCl, 1 mM $MgCl_2$, 0.1% BSA and 0.1% $NaN_3$] was added 50 µl of a hCG-containing fluid, followed by addition of one ball of a polystyrene ball which supports hCG specific antibody N305BS. The reaction was conducted at room temperature overnight. After completion of the reaction, the polystyrene ball was washed well with 0.01 M phosphate buffer (pH 7.0) and reacted with 200 µl of a β-D-galactosidase-anti-hCG rabbit antibody N305BG (Fab') conjugate at 37°C for 3 hours. After the above reaction, the polystyrene ball was thoroughly washed with 0.01 M phosphate buffer (pH 7.0) again and the β-D-galactosidase activity bound to the polystyrene ball was determined. Thus, 500 µl of 20 µl/ml of a solution of 4-methylumbelliferyl-β-D-galactoside was added to the polystyrene ball, the reaction was conducted at 37°C for one hour, the enzymatic reaction was then terminated by adding 3 ml of 0.1 mole of carbonate buffer (pH 10.5) and enzymatic activity was measured with a fluorometer (excitation at 365 nm., emission at 450 nm). The result is shown by the plotting -o- in Fig. 2.

### Example 2

An assay of hCG similar to that of Example 1, wherein polystyrene ball-hCG specific antibody N313BS obtained in Reference Example 5 (2) instead of polystyrene ball-hCG specific antibody N305BS in Example 1, was conducted.

The result is shown by the plotting -□- in Fig. 2.

### Example 3

An assay of hCG similar to that of Example 1, wherein polystyrene ball-hCG specific antibody N307BS obtained in Reference Example 6 instead of polystyrene ball-hCG specific antibody N305BS in Example 1, was conducted.

The result is shown by the plotting -▼- in Fig. 2.

### Reference Example 7

Quantitative determination of hCG with a β-D-galactosidase-anti-hCG rabbit antibody N305BS (IgG) conjugate:

(1) Preparation of a β-D-galactosidase-anti-hCG rabbit antibody N305BS (IgG) conjugate:

The specific antibody N305BS (2 mg) obtained in Reference Example 2 was reduced with 15 mM of β-mercaptoethylamine and, then, passed through a Sephadex G-25 column (0.9 cm in dia., 55 cm long) to remove the excess β-mercaptoethylamine. Then, 1.5 ml of a saturated solution of PDM was added and the mixture was reacted at 30°C for 30 minutes. The reaction mixture was passed through a Sephadex G-25 column (0.9 cm in dia., 55 cm long) to remove the excess PDM. To the resulting maleimidated antibody IgG was added 30 µl of a solution of β-D-galactosidase (5 mg/ml) and the mixture was reacted at 5°C for 36 hours. After completion of the reaction, the reaction mixture was purified by Sepharose 6B column chromatography using a 0.02 M phosphate buffer (pH 6.5) containing 0.1% BSA, 0.1% $NaN_3$, 1 mM $MgCl_2$ and 0.1 M NaCl to recover a fraction containing enzymatic activity and antibody activity. This was a β-D-galactosidase-anti-hCG antibody N305BS (IgG) conjugate.

Except for the following properties 5) and 8), the physical properties of this conjugate are identical with those of the conjugate according to Example 1 [properties 1), 2), 3), 4) and 6)].

5)  This conjugate has a molecular weight of about 800,000 and its molar antibody IgG/enzyme ratio is about 1.9.

7)  The ultraviolet absorption spectrum of the conjugate is shown in Fig. 3.

8)  This amino acid analysis of this conjugate is shown in Table 2.

Table 2

| Amino acid | β-D-Galactosidase-anti-hCG rabbit antibody N305BS (IgG) conjugate |
|---|---|
| Lys | 101 |
| Arg | 83 |
| Asp | 151 |
| Thr | 125 |
| Ser | 113 |
| Glu | 195 |
| Pro | 95 |
| Gly | 100 |
| Ala | 116 |
| Val | 114 |
| I-Leu | 52 |
| Leu | 142 |
| Tyr | 42 |
| Phe | 62 |

(Note): The number of moles of each amino acid per 100 moles of glycine.

(2) Quantitative determination of hCG with a β-D-galactosidase-anti-hCG rabbit antibody N305BS (IgG) conjugate:

The assay procedure of Example 1-(2) was repeated except that a polystyrene ball which supports hCG non-specific antibody N305BG and a β-D-galactosidase-anti-hCG rabbit antibody N305BS (IgG) conjugate were used in lieu of the polystyrene ball-hCG specific antibody N305BS and β-D-galactosidase-anti-hCG rabbit antibody N305BS (Fab') conjugate used in Example 1-(2). The result is shown by the plotting -∇- in Fig. 2.

Reference Example 8

Quantitative determination of hCG with a β-D-galactosidase-anti-hCG rabbit antibody N313BS (IgG) conjugate:

(1) Preparation of a β-D-galactosidase-anti-hCG rabbit antibody N313BS (IgG):

- 29 -

The procedure of Reference Example 7-(1) was repeated except that the N313BS prepared in Reference Example 3 was used in lieu of the N305BS prepared in Reference Example 7-(1) to give a β-D-galactosidase-anti-hCG rabbit antibody N313BS (IgG) conjugate.

Except for the following properties [ 5) and 8)], the physical properties of this conjugate were identical with those of the conjugate according to Example 1 [properties 1), 2), 3), 4), 6) and 7)].

5)  This conjugate has a molecular weight of about 800,000 and its molar antibody IgG/enzyme ratio is about 1.6.

8)  The amino acid analysis of this conjugate is shown in Table 3.

Table 3

| Amino acid | β-D-Galactosidase-anti hCG rabbit antiboty N313BS (IgG) conjugate |
|---|---|
| Lys | 105 |
| Arg | 84 |
| Asp | 155 |
| Thr | 122 |
| Ser | 115 |
| Glu | 192 |
| Pro | 88 |
| Gly | 100 |
| Ala | 115 |
| Val | 114 |
| I-Leu | 53 |
| Leu | 140 |
| Tyr | 41 |
| Phe | 60 |

(Note): The number of moles of each amino acid per 100 moles of glycine.

(2) Quantitative determination of hCG with a β-D-galactosidase-anti-hCG rabbit antibody N313BS (IgG) conjugate:

The procedure of Example 1-(2) was repeated except that a β-D-galactosidase-anti-hCG rabbit antibody N313BS (IgG) conjugate was used in lieu of the β-D-galactosidase-anti-hCG rabbit antibody N305BS (IgG) conjugate. The result is shown by the -●- plotting in Fig. 2.

Reference Example 9

Preparation of a β-D-galactosidase-anti-hCG antibody N305BG conjugate:

(1) In 2 ml of 0.05 M phosphate buffer (pH 7.0) was dissolved 4 mg of the antibody N305BG obtained in Reference Example 4, followed by the addition of 200 μl of m-maleimidobenzoyl-N-hydroxysuccinimide ester. The reaction was conducted at 30°C for 30 minutes. This reaction mixture was passed through a column of Sephadex G-25 (0.9 cm in dia., 55 cm long) equilibrated with 0.02 M phosphate NaCl buffer to separate the excess reagent from the maleimidated antibody. The resultant maleimidated antibody solution (0.5 ml) was added gradually to 0.3 ml of s solution (1 mg/ml) of β-D-galactosidase in 0.02 M phosphate buffer (pH 7.5). With occasional shaking, the mixture was reacted at 5°C overnight. After completion of the reaction, the reaction product was purified by Sepharose 6B column chromatography using 0.02 M phosphate-NaCl buffer (pH 7.0). The fractions containing enzymatic and antibody activities were recovered to give a β-D-galactosidase-anti-hCG antibody N305BG.

(2) To 5 mg of the antibody N305BG obtained in Reference Example 4 was added 0.1 mg of pepsin and the mixture was reacted at 30°C overnight. The reaction mixture was purified by means of a Sephadex G-150 column (2.5 cm in dia., 55 cm long). The resultant antibody $F(ab')_2$ fraction was reduced with β-mercaptoethylamine and passed through a Sephadex G-25 column (0.9 cm in dia., 55 cm long) to remove the excess reagent. Then, 2 ml of a saturated solution of N,N'-O-phenylenedimaleimide was added and the reaction was conducted at 30°C for 30 minutes. The reaction mixture was purified by means of a Sephadex G-25 column (2.5 cm in dia., 55 cm long). To the resultant maleimidated Fab' was added 50 μl of β-D-galactosidase solution (5 mg/ml) and the reaction was conducted at 5°C overnight. After completion of the reaction, the product was purified by Sepharose 6B column chromatography using a 0.02 M phosphate buffer (pH 6.5) containing 0.1% BSA, 0.1% NaN$_3$, 1 mM MgCl$_2$ and 0.1 M NaCl. The fractions containing enzymatic and antibody activities were recovered to give a β-D-galactosidase-labeled anti-hCG antibody conjugate.

## Reference Example 10

To 150 μl of Buffer A [a 0.02 M sodium phosphate buffer containing 0.1 M NaCl, 1 mM MgCl$_2$, 0.1% BSA and 0.1% NaN$_3$, pH 7.0] or Buffer B [a 0.02 M sodium phosphate buffer containing 0.15 M NaCl, 1 mM MgCl$_2$, 1% BSA, 0.1% NaN$_3$ and 10% sheep serum, pH 7.0] was added 50 μl of a solution of hCG or hLH in the corresponding buffer, followed by addition of one polystyrene ball which supports anti-hCG antibody N305BS obtained in Reference Example 5 (1). The mixture was reacted at room temperature overnight. After completion of the reaction, the polystyrene ball was washed well with 0.01 M phosphate NaCl buffer (pH 7.0) and, then, 200 μl of a solution of β-D-galactosidase-labeled anti-hCG antibody conjugate (obtained in Example 1-(1)) in Buffer A was added. The reaction was conducted at 37°C for 3 hours. After the reaction, the polystyrene ball was washed well with 0.01 M phosphate NaCl buffer (pH 7.0) again and the β-D-galactosidase activity bound to the polystyrene ball was measured. The results are shown in Fig. 4. In Fig. 4, ---●--- represents the non-specific adsorption of hLH in the Buffer A system, ---o--- represents the non-specific adsorption of hLH in the Buffer B system, ——●—— represents the standard curve of hCG obtained with Buffer A, and ——o—— represents the

standard curve of hCG obtained with Buffer B.

It will be apparent from the above results that as compared with the Buffer A system, the Buffer B system containing both a serum and a proteinous substance was only one-fourth as reactive to hLH while showing a comparable sensitivity toward hCG.

### Reference Example 11

To each of Buffer (1) [a 0.02 M sodium phosphate buffer containing 0.15 M NaCl, 0.5% ethylenediamine·tetraacetate·disodium·$2H_2O$ and 0.1% $NaN_3$; pH 7.0], Buffer (2) [a 0.02 M sodium phosphate buffer containing 0.15 M NaCl, 0.5% ethylenediamine·tetraacetate·disodium·$2H_2O$, 0.1% $NaN_3$ and 1% BSA; pH 7.0], Buffer (3) [a 0.02 M sodium phosphate buffer containing 0.15 M NaCl, 0.5% ethylenediamine·tetraacetate·disodium·$2H_2O$, 0.1% $NaN_3$ and 10% sheep serum; pH 7.0] and Buffer (4) [a 0.02 M sodium phosphate buffer containing 0.15 M NaCl, 0.5% ethylene-diamine·tetraacetate·disodium·$2H_2O$, 0.1% $NaN_3$, 1% BSA and 10% sheep serum; pH 7.0] was added 50 µl of a solution of hCG or hLH in the corresponding buffer, followed by addition of one polystyrene ball which supports anti-hCG antibody N313BS. The mixture was reacted at room temperature overnight. After completion of the reaction, the polystyrene ball was washed well with 0.01 M phosphate NaCl buffer (pH 7.0) and, then, 200 µl of the solution of β-D-galactosidase-labeled anti-hCG antibody conjugate in the Buffer A as used in Example 1 was added. The reaction was conducted at 37°C for 3 hours. After completion of the reaction, the polystyrene ball was washed well with 0.01 M phosphate NaCl buffer (pH 7.0) again and the β-D-galactosidase activity bound to the polystyrene ball was assayed. The results are shown in Table 4.

Table 4

| Buffer solution | Non-specific hLH adsorption (%) | | | |
| --- | --- | --- | --- | --- |
| | Concentration of hLH (mIU/tube) | | | |
| | 4.2 | 8.4 | 16.8 | 33.5 |
| (1) | 0.25 | 0.48 | 0.58 | 1.09 |
| (2) | 0.10 | 0.13 | 0.38 | 0.74 |
| (3) | 0.18 | 0.19 | 0.35 | 0.40 |
| (4) | 0.15 | 0.18 | 0.22 | 0.34 |

It will be apparent from Table 4 that as compared with the Buffer (1) system, the Buffer (4) system containing both a serum and a proteinous substance showed only about one-third of non-specific hLH adsorption.

Reference Example 12

A hCG specific immunochemical assay kit and the assay of hCG:

Using the hCG immunochemical assay kit described below, the plasma levels of hCG in healthy subjects, patients who had undergone ovariectomy and women in ovulatory phase were determined by the procedure described below.

hCG immunochemical assay kit:

(1) Polystyrene balls, 3.2 mm in dia., sensitized with 0.6 µg per ball of specific antibody as prepared in Reference Example 2,

(2) A portion of β-D-galactosidase-labeled anti-hCG antibody as prepared in Example 1 which contains about 100 µU of enzymatic activity,

(3) 0-100 mIU of standard hCG,

(4) As the diluent for the above reagents (1), (3) and the sample fluid, a 0.02 M phosphate buffer (pH 7.0) containing 10% normal sheep serum, 1% bovine serum albumin, 0.5% ethylenediamine·tetraacetate·disodium·2H$_2$O, 0.1% NaN$_3$ and 0.15 M NaCl,

(5)　20 µg of 4-methylumbellifery-β-D-galactoside,

(6)　As the diluent for the above reagent (2) and substrate (5), a 0.02 M phosphate buffer (pH 7.0) containing 0.1% bovine serum albumin, 0.1% $NaN_3$, 1 mM $MgCl_2$ and 0.1 M NaCl,

(7)　A 0.05 M phosphate buffer (pH 7.0) containing 0.1 M NaCl, and 1 mM $MgCl_2$ for washing the above polystyrene ball (1) and

(8)　A 0.1 M carbonate buffer at pH 10.5.

Procedure

To 50 µl of standard hCG or the sample fluid is added 150 µl of reagent (4) and one ball of reagent (1), and the reaction is carried out at room temperature overnight. After the reaction, the polystyrene ball is washed with reagent (7), and 200 µl of a dilution of reagent (2) in reagent (6) is added. The reaction is conducted at 37°C for 3 hours. The polystyrene ball is washed with reagent (7) again, and 500 µl of reagent (5) is added to the system for an enzymatic reaction. This reaction is conducted at 37°C for 1 hour, at the end of which time it was terminated with 2.5 ml of reagent (8). The fluorescence intensity of the reaction system is measured to estimate the concentration of hCG in the sample.

The plasma levels of hCG in healthy subjects, patients who had undergone ovariectomy and women in ovulatory phase were determined. The results are shown in Table 5. As control, the results found by the conventional non-specific enzyme-immunoassay are also shown.

Table 5

| Test sample | | Concentration of hCG (mIU/ml) | |
|---|---|---|---|
| | | HCG specific assay kit of invention (B) | Conventional kit |
| Healthy woman | 1 | 0.46 | 22.1 |
| | 2 | 0.40 | 15.5 |
| | 3 | 0.68 | 11.0 |
| | 4 | 0.66 | 12.2 |
| | 5 | 0.74 | 10.4 |
| Ovariectomized woman | 1 | 1.70 | 84.0 |
| | 2 | 0.64 | 77.7 |
| | 3 | 0.54 | 32.7 |
| | 4 | 2.99 | 80.7 |
| | 5 | 1.21 | 47.7 |
| Women in ovulatory phase | | 0.82 | 52.0 |

- 36 -

What is claimed is:

1.    In an immunochemical assay of human chorionic gonadotropin involving the use of an antibody supported on a carrier, an antigen and an antibody labeled with a labeling agent, an improvement comprises in that said supported antibody and the labeled antibody are dissimilar antibodies which are not overlapping with each other in antigen-determinant position, that the supported antibody is an antibody specifically reactive to human chorionic gonado-tropoin, that said labeling agent is β-D-galactosidase, and that said β-D-galactosidase is coupled with the corresponding antibody employing N,N'-O-phenylenedimaleimide.

2.    An improvement as claimed in Claim 1, wherein the antibody specifically reactive to human chorionic gonadotropin is an antibody obtained by contacting a body fluid containing an anti-hCG antibody with a peptide of the formula:

　　　H-R-Pro-Ser-Asp-Thr-Pro-Ile-Leu-Pro-Gln-OH [wherein R is a partial peptide of 1 to 14 amino acid residues including 14-Gly of the peptide $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$],

as immobilized on a carrier, and separating the anti-hCG antibody specifically absorbed by elution.

3.    An improvement as claimed in Claim 2, wherein the peptide is H-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

4.    An improvement as claimed in Claim 1, wehrein the antibody specifically reactive to human chorionic gonadotropin is an antibody obtained by conjugating a peptide of the formula:

　　　H-R-Pro-Ser-Asp-Thr-Pro-Ile-Leu-Pro-Gln-OH [wherein R is a partial peptide of 1 to 14 amino acid residues including 14-Gly of the peptide $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$]
with a carrier protein in the presence of glutaraldehyde and inoculating the resulting conjugate into a warm-blooded animal other than man to produce an antibody and recovering it.

5.    An improvement as claimed in Claim 4, wherein the peptide is H-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

6.    An immunochemical assay kit for use in an assay of human chorionic gonadotropin, which comprises
   1)    a reagent consisting of a conjugate which is prepared by coupling β-D-galactosidase with an antibody employing N,N'-O-phenylenedimaleimide and
   2)    an antibody supported on a carrier, the antibody being not overlapping in antigen-determinant position with the antibody to be coupled with β-D-galactosidase and being specifically reactive to human chorionic gonadotropin.

7.    An immunochemical assay kit as claimed in Claim 6, wherein the antibody specifically reactive to human chorionic gonadotropin is an antibody obtained by containing a body fluid containing an anti-hCG antibody with a peptide of the formula:
       H-R-Pro-Ser-Asp-Thr-Pro-Ile-Leu-Pro-Gln-OH
[wherein R is a partial peptide of 1 to 14 amino acid residues including 14-Gly of the peptide $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$].
as immobilized on a carrier, and separating the anti-hCG antibody specifically absorbed by elution.

8.    An immunochemical assay kit as claimed in Claim 7, wherein the peptide is H-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.


9.    An immunochemical assay kit as claimed in Claim 6, wherein the antibody specifically reactive to human chorionic gonadotropin is an antibody obtained by conjugating a peptide of the formula:

H-R-Pro-Ser-Asp-Thr-Pro-Ile-Leu-Pro-Gln-OH [wherein R is a partial peptide of 1 to 14 amino acid residues including 14-Gly of the peptide $Ala^1$-$Pro^2$-$Pro^3$-$Pro^4$-$Ser^5$-$Leu^6$-$Pro^7$-$Ser^8$-$Pro^9$-$Ser^{10}$-$Arg^{11}$-$Leu^{12}$-$Pro^{13}$-$Gly^{14}$]

with a carrier protein in the presence of glutaraldehyde and inoculating the resulting conjugate into a warm-blooded animal other than man to produce an antibody and recovering it.


10.    An immunochemical assay kit as claimed in Claim 9, wherein the peptide is H-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln-OH.

0088368

Fig. 1

Wave length (nm)

Fig. 2

hCG (mIU)

Fig. 3

Fig. 4